(19) Europäisches Patentamt
European Patent Office
Office européen des brevets

(11) Veröffentlichungsnummer: **0 112 541**
**A1**

(12) # EUROPÄISCHE PATENTANMELDUNG

(21) Anmeldenummer: **83112685.9**

(22) Anmeldetag: **16.12.83**

(51) Int. Cl.³: **C 07 D 209/48**, C 07 D 207/40

(30) Priorität: **28.12.82 DE 3248331**

(43) Veröffentlichungstag der Anmeldung: **04.07.84**
**Patentblatt 84/27**

(84) Benannte Vertragsstaaten: **CH DE FR GB LI**

(71) Anmelder: **BAYER AG, Konzernverwaltung RP**
**Patentabteilung, D-5090 Leverkusen 1 Bayerwerk (DE)**

(72) Erfinder: **Halcour, Kurt, Dr., Gellertstrasse 14,**
**D-5090 Leverkusen 1 (DE)**
Erfinder: **Lange, Peter-Michael, Dr.,**
**Walter-Flex-Strasse 9, D-5090 Leverkusen 1 (DE)**
Erfinder: **Wagner, Rudolf, Dr., Carl-Duisberg-Platz 21,**
**D-5090 Leverkusen 1 (DE)**

(54) **SO3/Imid-Addukte, Verfahren zu ihrer Herstellung und ihre Verwendung als Sulfonierungsmittel.**

(57) Die Erfindung betrifft $SO_3$-Addukte von in Wasser schwer bzw. unlöslichen Imiden der Formel

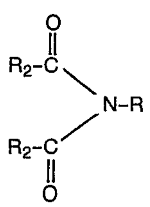

(I)

in der $R_1$, $R_2$ und R die in der Beschreibung angegebene Bedeutung haben, ein Verfahren zur Herstellung der Addukte und ihre Verwendung als Sulfonierungsmittel.

EP 0 112 541 A1

BAYER AKTIENGESELLSCHAFT 5090 Leverkusen, Bayerwerk
Zentralbereich
Patente, Marken und Lizenzen B/bo/bc/c

# $SO_3$/Imid-Addukte, Verfahren zu ihrer Herstellung und ihre Verwendung als Sulfonierungsmittel

Die Erfindung betrifft neue $SO_3$-Addukte, deren Herstellung und ihre Verwendung als Sulfonierungsmittel.

Es ist bekannt, daß $SO_3$ sich an Adduktbildner anlagern kann und daß man diese $SO_3$-Addukte als Sulfonierungsmittel verwenden kann (s. z.B. die Übersichtsarbeit von E.E. Gilbert in Chemical Reviews, Vol. 62, 1962, Seiten 550 ff). Als Adduktbildner wurden tertiäre Amine, tertiäre Amide, Stickstoff- und/oder Sauerstoff- und/oder Schwefel-haltige Heterocyclen, ferner Ether oder auch Benzoesäure verwendet.

Diese bekannten $SO_3$-Addukte haben den Nachteil, daß in ihnen das $SO_3$ vielfach so stark desaktiviert ist, daß seine Reaktionsfähigkeit nicht mehr zum Sulfonieren schwerer sulfonierbarer Verbindungen ausreicht. Weiterhin sind die Adduktbildner nach erfolgter Sulfonierung, wenn überhaupt, nur mit erheblichen Verlusten zurückgewinnbar. Infolge dieser unvollständigen Rückgewinnbarkeit sind die Sulfonierungen zum einen kostspielig und führen zum anderen zu einer Belastung des Abwassers mit Adduktbildnern.

Es wurde nun gefunden, daß man zu $SO_3$-Addukten mit wesentlich verbesserten Eigenschaften gelangt, die die vorstehend aufgezeigten Nachteile nicht mehr aufweisen, wenn man anstelle der bislang verwendeten Adduktbildner nunmehr bestimmte Imide als Adduktbildner einsetzt.

Die Erfindung betrifft $SO_3$-Addukte von in Wasser schwer bzw. unlöslichen Imiden der Formel

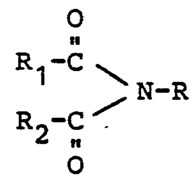

(I)

in der

$R_1$ und $R_2$ unabhängig voneinander für einen gegebenenfalls substituierten Phenylrest stehen oder zusammen einen gegebenenfalls substituierten Phenylen-1,2-Rest, einen Alkylenrest der Formel $-(CH_2)_m-$, in der m 2, 3 oder 4 ist, oder einen Ethenylenrest der Formel $-\underset{R_3}{C} = \underset{R_4}{C}-$ bilden,
in dem $R_3$ und $R_4$ unabhängig voneinander Wasserstoff, Halogen oder $C_1$-$C_4$-Alkyl bedeuten oder zusammen einen Butylen-1,4-Rest bilden; und

R für einen gegebenenfalls substituierten Alkyl-, Cycloalkyl-, Benzyl- oder Phenylrest, einen sich von einer Monocarbonsäure ableitenden Acylrest oder einen Rest der Formel

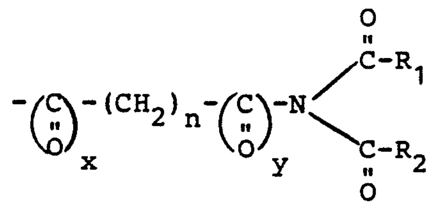

steht, in der $R_1$ und $R_2$ die vorstehend angegebene Bedeutung haben, x und y unabhängig voneinander für 0 oder 1 stehen und n die Werte 0, 2, 3, 4, 5 oder 6 annehmen kann, mit der Maßgabe, daß x, y und n nicht gleichzeitig 0 sein dürfen und daß x und y gleich sein müssen, wenn n 2, 3, 4, 5 oder 6 ist.

Die Addukte enthalten je Imid-Gruppe 1 bis 3 Moleküle $SO_3$.

Die erfindungsgemäßen $SO_3$/Imid-Addukte zeichnen sich dadurch aus, daß sie mehr $SO_3$ enthalten können (bis zu 3 Mol $SO_3$ je Mol Imidgruppe), daß das $SO_3$ in den Addukten so reaktiv ist, daß es auch noch schwierig zu sulfonierende Verbindungen wie Benzol, Biphenyl, Naphthalin oder Polystyrole zu sulfonieren vermag und daß die Addukte trotz ihrer hohen Reaktionsfähigkeit noch eine, verglichen mit der Selektivität von $SO_3$, wesentlich verbesserte Selektivität aufweisen.

Da die erfindungsgemäßen Adduktbildner in Wasser schwer löslich bzw. unlöslich sind, können sie ohne Schwierigkeiten verlustlos zurückgewonnen werden. Die zurückgewonnenen Adduktbildner können anschließend wiederverwendet werden. Die mit Hilfe der erfindungsgemäßen $SO_3$/Imid-

Addukte durchgeführten Sulfonierungen sind deshalb nicht nur wirtschaftlicher als die mit den bekannten $SO_3$-Addukten durchgeführten Sulfonierungen, sondern auch umweltfreundlicher, da die erfindungsgemäßen Adduktbildner das Abwasser nicht belasten.

Als Substituenten für die Phenylreste bzw. den Phenylen-1,2-Rest von $R_1$ und $R_2$ und den Benzyl- und den Phenylrest von R kommen vor allem solche in Betracht, die inert gegenüber $SO_3$ sind. Es ist vorteilhaft, wenn diese Substituenten weiterhin die Reaktionsfähigkeit des Benzolrings herabsetzen. Solche Substituenten sind zum Beispiel niedere Alkylgruppen wie die Methylgruppe; niedere Halogenalkylgruppen wie die Trifluormethylgruppe; Halogenatome wie Fluor- und Chloratome; ferner die Nitrogruppe.

Für $R_1$, $R_2$ und R seien als gegebenenfalls substituierte Phenylreste beispielsweise genannt: der Phenyl-, Tolyl-, Xylyl-, Trifluormethylphenyl-, Chlorphenyl-, Dichlorphenyl-, Fluorphenyl- und der Nitrophenylrest.

Als gegebenenfalls substituierte Phenylen-1,2-Reste seien für $R_1$ und $R_2$ genannt z.B. der Mono- und Dichlorphenylen-1,2-Rest; der Mono- und Difluorphenylen-1,2-Rest und der Mono- und Dinitrophenylen-1,2-Rest.

Für R seien als gegebenenfalls substituierte Benzylreste beispielsweise genannt: der Benzyl-, $\alpha$-Methylbenzyl-, der 2-, 3- oder 4-Methylbenzyl-, Chlorbenzyl-, Dichlorbenzyl-, Trifluormethylbenzyl- und der Sulfobenzyl-Rest. Der Benzylrest kann auch in 4-Stellung an eine vernetzte Polyethylenkette gebunden sein. Imide der Formel (I),

in denen R für einen solchen an eine vernetzte Polyethylenkette gebundenen Benzylrest steht, sind hochmolekulare unlösliche Verbindungen.

Für R seien als gegebenenfalls substituierte Alkylreste beispielsweise genannt: $C_1$-$C_{12}$-Alkylreste wie der Methyl-, Ethyl-, Propyl-, Butyl-, Pentyl-, Hexyl-, 2-Ethylhexyl-Rest und halogenierte, vorzugsweise chlorierte und/oder fluorierte $C_1$-$C_{12}$-Alkylreste wie der Chlormethyl-, Dichlormethyl-, Trichlormethyl-, Fluormethyl-, Difluormethyl-, Trifluormethyl-Rest.

Für R seien als gegebenenfalls substituierte Cycloalkylreste beispielsweise genannt: $C_5$-$C_{12}$-Cycloalkylreste wie der Cyclohexyl-, Methylcyclohexyl-, Dimethylcyclohexyl-, tert.-Butylcyclohexyl-Rest und halogenierte, vorzugsweise chlorierte und/oder fluorierte $C_5$-$C_{12}$-Cycloalkylreste wie der Chlorcyclohexyl-, Dichlorcyclohexyl- und der Trichlormethylcyclohexylrest.

Für R seien als Acylreste von Monocarbonsäuren vor allem Acylreste aliphatischer $C_1$-$C_4$-Monocarbonsäuren, z.B. der Formyl-, Acetyl- oder Propionylrest, und als Acylrest aromatischer Monocarbonsäuren z.B. der Benzoylrest genannt.

Die erfindungsgemäßen $SO_3$/Imid-Addukte können sowohl monomolekulare als auch hochmolekulare Verbindungen sein, je nach dem, ob das als Adduktbildner verwendete Imid monomolekular oder hochmolekular ist.

Die erfindungsgemäßen monomolekularen $SO_3$/Imid-Addukte (Typ A der erfindungsgemäßen $SO_3$/Imid-Addukte) sind in organischen Lösungsmitteln wie sie für die Sulfonierung

mit $SO_3$ üblicher Weise verwendet werden, löslich und ermöglichen deshalb eine Sulfonierung in homogener Phase. Den monomolekularen $SO_3$/Imid-Addukten liegen als Adduktbildner Imide der vorstehend angegebenen Formel (I) zugrunde, in der $R_1$, $R_2$ und R die unter Formel (I) angegebene Bedeutung haben mit der Maßgabe, daß R nicht für einen gegebenenfalls substituierten Benzylrest stehen darf, der an eine vernetzte Polyethylenkette gebunden ist. Die erfindungsgemäßen monomolekularen $SO_3$/Imid-Addukte enthalten vorzugsweise 1 bis 2 Moleküle $SO_3$ je Imidgruppe.

Bevorzugt sind solche $SO_3$/Imid-Addukte des Typs A, denen als Adduktbildner Imide der Formel (I) zugrundeliegen, in der R für eine $C_1$-$C_4$-Alkyl-, eine Cyclohexyl-, eine $R_1$-CO-N-$(CH_2)_2$- (mit $R_2$-CO/ am N) oder eine $R_1$-CO-N-CO-Gruppe (mit $R_2$-CO/ am N) steht.

Die erfindungsgemäßen hochmolekularen $SO_3$/Imid-Addukte (Typ B der erfindungsgemäßen $SO_3$/Imid-Addukte) sind in allen Lösungsmitteln unlöslich. Sie dienen deshalb für Sulfonierungen in heterogener Phase.

Den erfindungsgemäßen hochmolekularen $SO_3$/Imid-Addukten liegen als Adduktbildner Imide der Formel (I) zugrunde, in der $R_1$ und $R_2$ die unter Formel (I) angegebene Bedeutung haben und R für einen gegebenenfalls substituierten Benzylrest steht, der an eine vernetzte Polyethylenkette gebunden ist. Die hochmolekularen $SO_3$/Imid-Addukte enthalten 1 bis 3, vorzugsweise 3 Moleküle $SO_3$ je Imidgruppe.

Bevorzugt sind wegen ihrer leichten Zugänglichkeit solche $SO_3$/Imid-Addukte des Typs B, denen als Adduktbildner Imide der Formel (I) zugrundeliegen, in der $R_1$ und $R_2$ einen gegebenenfalls substituierten Phenylen-1,2-Rest bilden und R für einen gegebenenfalls substituierten Benzylrest steht, der an eine vernetzte Polyethylenkette gebunden ist. Die bevorzugten Imide des Typs B fallen als Zwischenprodukte bei der Herstellung von Anionenaustauschern durch Amidomethylierung von perlförmigen vernetzten Polystyrolen an (s. Ullmanns Enzyklopädie der technischen Chemie, 14. Auflage, Band 13, Seite 302). Als Adduktbildner für die bevorzugten $SO_3$/Imid-Addukte des Typs B werden Imidoalkylierungsprodukte von makroporösen Polystyrol-Perlpolymerisaten verwendet, die mit 3 bis 12 Gew.-% eines Vernetzungsmittels, z.B. Divinylbenzol, vernetzt wurden. Vorzugsweise werden Imidoalkylierungsprodukte solcher vernetzten makroporösen Polystyrol-Perlpolymerisate eingesetzt, die unter Verwendung von etwa 40 bis 100 Gew.-%, vorzugsweise 50 bis 90 Gew.-%, bezogen auf das Gesamtgemisch der Monomeren, einer porenbildenden Komponente (Fäll- oder Quellungsmittel für das Polymer) hergestellt wurden.

Die Erfindung betrifft ferner ein Verfahren zur Herstellung der erfindungsgemäßen $SO_3$/Imid-Addukte. Das Verfahren besteht darin, daß man die Imide der Formel (I) in Gegenwart von gegen $SO_3$ inerten organischen Lösungsmitteln mit der für die gewünschte Beladung erforderlichen $SO_3$-Menge, 1 bis 3 Molen $SO_3$ je Mol Imidgruppe, bei Temperaturen von 0 bis 80°C, vorzugsweise 20 bis 60°C, umsetzt.

Als gegen $SO_3$ inerte organische Lösungsmittel haben sich Halogenkohlenwasserstoffe, insbesondere aliphatische Halogenkohlenwasserstoffe wie Methylenchlorid, Chloroform, Tetrachlorkohlenstoff, 1,2-Dichlorethan, 1,1,2-Trichlorethylen und 1,1,2,2-Tetrachlorethan bewährt. Vorzugsweise werden 1,2-Dichlorethan und Methylenchlorid verwendet.

Die organischen Lösungsmittel werden in einer solchen Menge eingesetzt, daß gut rührbare und gut kühlbare Lösungen bzw. Suspensionen der Imide der Formel (I) entstehen; im allgemeinen hat es sich bewährt, wenn auf 1 Gew.-Teil Imid 1 bis 20, vorzugsweise 5 bis 10, Gew.-Teile Lösungsmittel entfallen.

Die Addition des $SO_3$ an die Imide der Formel (I) verläuft sehr glatt und schnell. Im allgemeinen ist die Adduktbildung innerhalb von 0,5 bis 3 Stunden nach Zusammengeben von Imid und $SO_3$ beendet. Die erfindungsgemäßen Addukte können aus der Reaktionslösung entweder mechanisch, z.B. durch Abfiltrieren oder durch Dekantieren der Reaktionslösung, abgetrennt oder durch Abdestillieren des organischen Lösungsmittels gewonnen werden. Nach vorsichtigem Waschen mit frischem organischem Lösungsmittel fallen die erfindungsgemäßen $SO_3$/Imid-Addukte in Form grauweißer Pulver (Typ A), oder in Form weißgrauer Perlpolymerisate (Typ B) an. Die Charakterisierung der erfindungsgemäßen $SO_3$/Imid-Addukte erfolgt allgemein durch Elementaranalyse, insbesondere Bestimmung des Schwefelwertes, und durch Titration des gebundenen $SO_3$.

Die erfindungsgemäßen $SO_3$/Imid-Addukte des Typs A sind ferner dadurch charakterisiert, daß ihr $^1$H-NMR-Spektrum gegenüber dem $^1$H-NMR-Spektrum des zugrundeliegenden Imids

im magnetischen Feld verschoben ist. Die für die Herstellung der erfindungsgemäßen $SO_3$/Imid-Addukte erforderlichen Imide der Formel (I) und deren Herstellung sind bekannt (s. Sachs, Chem.Ber. 31, 1228 und Evans, Dehn. J.Amer.Chem.Soc. 51, S. 3652 für die Herstellung der monomolekularen Imide und Ullmanns Enzyklopädie der technischen Chemie, 14. Auflage, Band 13, Seite 302 für die Herstellung der hochmolekularen Imide).

Für die Verwendung als Sulfonierungsmittel ist die Isolierung der $SO_3$/Imid-Addukte in den meisten Fällen nicht erforderlich; vielmehr können die erfindungsgemäßen $SO_3$/Imid-Addukte in Form ihrer Lösungen in den organischen, gegen $SO_3$ inerten Lösungsmitteln unmittelbar für die Sulfonierung verwendet werden.

Die Erfindung betrifft ferner die Verwendung der erfindungsgemäßen $SO_3$/Imid-Addukte als Sulfonierungsmittel. Das erfindungsgemäße Verfahren zur Sulfonierung von organischen Verbindungen mit Hilfe der erfindungsgemäßen $SO_3$/Imid-Addukte eignet sich insbesondere zur Sulfonierung von aromatischen Kohlenwasserstoffen, wie Benzol, Naphthalin, Anthracen, Biphenyl, Diphenylalkanen und Alkylaromaten, wie Toluol, Xylol, Dodecylbenzol oder substituierten aromatischen Kohlenwasserstoffen, wie Halogenaromaten, Phenolen, Phenolethern, Diphenylether, Diphenylthioether, aromatischen Aminen, N-alkylierten aromatischen Aminen, multifunktionell substituierten aromatischen Verbindungen, wie Aminophenolen und Aminophenolethern.

Bei den Diphenylderivaten (wie Biphenyl, Diphenylalkanen, Diphenylether) kann die Sulfonierung mit den erfindungsgemäßen $SO_3$/Imid-Addukten wahlweise zu Mono- oder Disulfonsäuren (z.B. 4,4'-Disulfonsäuren) führen, die nach bekannten Verfahren in die entsprechenden Amino- oder Hydroxyverbindungen übergeführt werden können.

Die erfindungsgemäßen Sulfonierungen werden allgemein in der Weise ausgeführt, daß man die zu sulfonierende Verbindung in eine Lösung oder Suspension des $SO_3$/Imid-Komplexes gibt und dieses Gemisch bei 0 bis 80°C, vorzugsweise bei 20 bis 30°C, 30 Minuten bis 5 Stunden rührt. Anschließend wird das Reaktionsgemisch aufgearbeitet.

Bei Verwendung der monomolekularen $SO_3$/Imid-Addukte hat sich für die Aufarbeitung folgende Arbeitsweise besonders bewährt:

Die Reaktionslösungen werden mit Wasser extrahiert. Zur Isolierung der gebildeten Sulfonsäuren aus den wäßrigen Extrakten werden die wäßrigen Lösungen, gegebenenfalls nach Neutralisation mit üblichen Basen, z.B. Alkalien oder Erdalkalien, zur Trockne eingeengt.

Die nach der Extraktion mit Wasser zurückbleibenden organischen Phasen enthalten das als Adduktbildner verwendete Imid und gegebenenfalls nicht sulfonierte Ausgangsverbindung.

Zur Rückgewinnung des Imids wird das organische Lösungsmittel abdestilliert und das als Rückstand verbleibende Gemisch aus Imid und Ausgangsverbindung nach bekannten Methoden, z.B. durch Destillation, in Imid und Ausgangsverbindung aufgetrennt. Das Imid kann anschließend erneut zur Herstellung des $SO_3$/Imid-Adduktes wiederverwendet werden.

Da die Sulfonierung mit Hilfe der erfindungsgemäßen $SO_3$/Imid-Addukte praktisch vollständig verläuft und deshalb die Mengen an unsulfonierter Verbindung vernachlässigbar gering sind, kann man in den meisten Fällen auf die Aufarbeitung der organischen Phase verzichten und die organische Phase unmittelbar nach Entfernen des in ihr verbliebenen Wassers erneut zur Herstellung des $SO_3$/Imid-Adduktes und anschließend an die $SO_3$/Imid-Addukt-Bildung wiederum zur Sulfonierung verwenden. Das heißt, die organische Phase und das in ihr enthaltene Imid werden im Kreislauf geführt. In Phase 1 des Kreislaufs (Herstellung des $SO_3$/Imid-Adduktes) wird das Imid mit $SO_3$ beladen, in Phase 2 des Kreislaufs (Sulfonierung) gibt das Imid das aufgenommene $SO_3$ an die zu sulfonierende Verbindung ab. Bei einer solchen Kreislaufführung läßt sich eine eventuelle Anreicherung der organischen Phase an Verunreinigungen dadurch vermeiden, daß man dieser einen Teilstrom entnimmt und reinigt und eine der entnommenen Menge an organischem Lösungsmittel und Imid entsprechende Menge an reinem Lösungsmittel und Imid in die organische Phase zurückleitet.

Bei Verwendung der hochmolekularen $SO_3$/Imid-Addukte hat sich für die Aufarbeitung folgende Arbeitsweise besonders bewährt:

Nach beendeter Sulfonierung wird das hochmolekulare Imid mechanisch von der Reaktionslösung abgetrennt, gegebenenfalls mit Lösungsmittel abgespült und anschließend erneut zur Adduktbildung wiederverwendet.

Die nach dem Abtrennen des Imids verbleibende Reaktionslösung wird zur Abtrennung der Sulfonsäuren mit Wasser extrahiert. Die Sulfonsäuren werden aus den

wäßrigen Extrakten wie vorstehend beschrieben als solche oder in Form ihrer Salze isoliert.

Das nach der Extraktion der Sulfonsäuren aus den Reaktionslösungen zurückbleibende organische Lösungsmittel wird nach Entfernen des Wassers abdestilliert und wieder eingesetzt oder aber nur getrocknet und unmittelbar wiederverwendet.

Entfällt in den erfindungsgemäßen $SO_3$/Imid-Addukten mehr als 1 Molekül $SO_3$ auf eine Imidgruppe, so sind die einzelnen $SO_3$-Moleküle mit unterschiedlicher Festigkeit an die Imidgruppe gebunden. Am festesten wird das erste, am losesten das dritte $SO_3$-Molekül an die Imidgruppe gebunden. Für besonders selektive Sulfonierungen wird man daher vorzugsweise solche $SO_3$/Imid-Addukte verwenden, die nur 1 Molekül $SO_3$ je Imidgruppe enthalten.

Beispiel 1

16,1 g (0,1 Mol) N-Methylphthalimid und 4,2 ml (0,1 Mol) Schwefeltrioxid werden in 50 ml Dichlormethan unter Rühren und Kühlen bei 0 bis +20°C vermischt. Es bildet sich ein weißer, flockiger Niederschlag. Anschließend wird das Dichlormethan im Vakuum bei 20°C abdestilliert.

Es werden 23,5 g (98 % der Theorie) des $SO_3$/N-Methylphthalimid-1:1-Adduktes in Form eines festen weißen Rückstandes erhalten. Das Addukt ist hygroskopisch und zerfließt langsam beim Stehen an der Luft.

In gleicher Weise wie das vorstehend beschriebene $SO_3$/N-Methylphthalimid-1:1-Addukt wurden auch die 1:1-Addukte von $SO_3$ an N-Ethylphthalimid, N-n-Propylphthalimid und N-sek.-Butylphthalimid hergestellt.

Die Ausbeuten an diesen 1:1-Addukten betrugen ebenfalls etwa 98 % der Theorie. Alle drei Produkte fielen ebenfalls in Form weißer, hygroskopischer Feststoffe an.

Beispiel 2

25,1 g (0,1 Mol) N-Benzoylphthalimid und 4,2 ml (0,1 Mol) Schwefeltrioxid werden wie in Beispiel 1 beschrieben miteinander umgesetzt. Es entsteht ein gelber flockiger Niederschlag. Nach dem Abdestillieren des Dichlormethans werden 31,4 g (96 % der Theorie) des $SO_3$/N-Benzoylphthalimid-1:1-Adduktes in Form eines gelben Feststoff erhalten. Das Addukt ist hygroskopisch und zerfließt beim Stehen an der Luft.

Beispiel 3

14,1 g (0,1 Mol) N-n-Propylsuccinimid und 4,2 ml (0,1 Mol) Schwefeltrioxid werden wie in Beispiel 1 beschrieben miteinander umgesetzt. Es entsteht ein rosa gefärbter Niederschlag. Nach dem Abdestillieren des Dichlormethans werden 21 g (97 % der Theorie) des $SO_3$/N-n-Propylsuccinimid-1:1-Adduktes in Form eines schwach rosa gefärbten Feststoffes erhalten.

Beispiel 4

16 g (0,1 Mol) N-Methylphthalimid werden mit einer Lösung von 13,3 ml (0,3 Mol) Schwefeltrioxid in 200 ml Dichlormethan unter Rühren und Kühlen bei 0 bis 20°C vermischt. Anschließend wird das Dichlormethan abdestilliert und der Rückstand auf 80°C erhitzt, um nicht gebundenes Schwefeltrioxid zu entfernen.

Nach dem Abkühlen auf Raumtemperatur werden 32 g (97 % der Theorie) $SO_3$/N-Methylphthalimid-2:1-Addukt in Form eines gelben kristallinen Feststoffs erhalten. Das 2:1-Addukt ist stark hygroskopisch und zerfließt beim Stehen an der Luft.

Der Schwefelgehalt des Produktes beträgt 19,9 % (theoretischer Wert: 20,0 %).

Beispiel 5

60 g (0,21 Mol) eines Phthalimido-methylierten, mit 8 Gew.-% Divinylbenzol vernetzten und mit 80 Gew.-% Isododecan makroporös gemachten Polystyrol-Perlpolymerisates (Anzahl der Phthalimidomethylgruppen je aromatischen Kerns im Polystyrol: 0,9) werden unter Rühren und Kühlen mit einer Lösung von 37 ml (0,88 Mol) Schwefeltrioxid in 200 ml Dichlormethan vermischt. Das Gemisch wird 2 Stunden bei 15 bis 30°C gerührt. Anschließend wird das mit $SO_3$-beladene Perlpolymerisat abfiltriert, mit etwas Dichlormethan abgespült und im Vakuum bei Raumtemperatur getrocknet.

Es werden 130 g $SO_3$/Imidharz-3:1-Addukt erhalten.

Die Analyse dieses $SO_3$/Imidharz-3:1-Adduktes ergibt, daß das Phthalimido-methylierte Polystyrol durch die Behandlung mit Schwefeltrioxid zum einen sulfoniert wurde (Anzahl der Sulfonsäuregruppen je aromatischen Kerns im Polystyrol: 0,9) und zum anderen 3 Moleküle $SO_3$ je Imidgruppe gebunden enthält.

Wurde anstelle des verwendeten Phthalimido-methylierten Polystyrol-Perlpolymerisates die gleiche Menge eines Phthalimido-methylierten, mit 5 Gew.-% Divinylbenzol vernetzten und mit 60 Gew.-% Isododecan makroporös gemachten Polystyrol-Perlpolymerisates oder eines Phthalimido-methylierten mit 6 Gew.-% Divinylbenzol vernetzten und mit 70 Gew.-% Isododecan makroporös gemachten Polystyrol-Perlpolymerisates eingesetzt (die Anzahl der Phthalimidogruppen je aromatischen Kerns betrug bei beiden Harzen etwa 0,9), so wurden $SO_3$/Imidharz-3:1-Addukte mit fast gleichen Eigenschaften erhalten.

Beispiel 6

32 g (0,2 Mol) N-Methylphthalimid werden mit einer Lösung von 8,3 ml (0,2 Mol) Schwefeltrioxid in 200 ml 1,2-Dichlorethan unter Rühren und Kühlen bei 0 bis 20°C vermischt. Die entstandene Lösung wird unter Rühren und Kühlen bei 0 bis 20°C tropfenweise mit einer Lösung von 25 g (0,2 Mol) Naphthalin in 50 ml 1,2-Dichlorethan versetzt. Die klare Lösung wird 1 Stunde bei 80°C (Rückflußtemperatur) gerührt.

Anschließend wird die Reaktionslösung 3 mal mit je 100ml Wasser extrahiert. Die vereinigten wäßrigen Extrakte werden mit 32 g 25 %iger wäßriger Natronlauge neutralisiert und anschließend im Rotationsverdampfer zur Trockne eingeengt. Es werden 45,5 g (99 % der Theorie) Naphthalin-1-sulfonsäure (Natriumsalz) in Form eines weißen Pulvers erhalten.

Die nach der Extraktion mit Wasser zurückbleibende Dichlorethanlösung wird im Rotationsverdampfer zur Trockne eingeengt. Es werden 31,5 g (98 % der eingesetzten Menge) N-Methylphthalimid zurückerhalten.

Bei der Sulfonierung von 0,2 Mol Naphthalin mit 0,2 Mol freiem Schwefeltrioxid wurden nur 55 % der Theorie Naphthalin-1-sulfonsäure (Natriumsalz) erhalten, dagegen als Nebenprodukte 21 % der Theorie Naphthalindisulfonsäure (Natriumsalz) und 12 % der Theorie Dinaphthylsulfon.

0112541

In der für die Sulfonierung von Naphthalin mit dem $SO_3$/N-Methylphthalimid-Addukt beschriebenen Weise wurden

a) Naphthalin mit dem 1:1-Addukt von $SO_3$ an N-Butylphthalimid,

b) Biphenyl mit dem 1:1-Addukt von $SO_3$ an N-Methylphthalimid und

c) n-Decanol mit dem 1:1-Addukt von $SO_3$ an N-Methylphthalimid

sulfoniert.

Dabei wurden

im Fall a) 89 g (97 % der Theorie) Naphthalin-1-sulfonsäure (Natriumsalz) erhalten und 56 g (98,9 %)des verwendeten N-Methylphthalimids zurückgewonnen.

Im Fall b) 40 g (78 % der Theorie) Biphenyl-4-sulfonsäure (Natriumsalz) erhalten und 32 g (100 %) des Adduktbildners N-Methylphthalimid zurückgewonnen.

Im Fall c) 52 g (100 % der Theorie) Dodecanolschwefelsäureester (Natriumsalz) erhalten und 31 g (97 %) des N-Methylphthalimids zurückgewonnen.

<u>Beispiel 7</u>

25,1 g (0,1 Mol) N-Benzoylphthalimid werden mit einer Lösung von 4,2 ml (0,1 Mol) Schwefeltrioxid in 100 ml

1,2-Dichlorethan unter Rühren und Kühlen bei 0 bis +20°C vermischt. Die Lösung wird unter Rühren und Kühlen bei 0 bis 20°C tropfenweise mit 7,8 g (0,1 Mol) Benzol versetzt.

Anschließend wird die Reaktionslösung wie im Beispiel 6 beschrieben aufgearbeitet.

Es werden 17,1 g (95 % der Theorie) Benzolsulfonsäure (Natriumsalz) in Form eines weißen Pulvers erhalten.

Es werden 24 g (96 %) N-Benzoylphthalimid zurückgewonnen.

Beispiel 8

14 g (0,1 Mol) N-Propylsuccinimid werden mit einer Lösung von 4,2 ml (0,1 Mol) Schwefeltrioxid in 100 ml 1,2-Dichlorethan unter Rühren und Kühlen bei 0 bis 20°C vermischt. In die Lösung werden unter Rühren und Kühlen bei 0 bis 20°C 15 g (0,1 Mol) Biphenyl eingetragen.

Die Reaktionslösung wird wie im Beispiel 1 beschrieben aufgearbeitet. Es werden 23,1 g (90 % der Theorie) Biphenyl-4-sulfonsäure (Natriumsalz) in Form eines weißen Pulvers erhalten und 13,5 g (96,5 %) N-Propylsuccinimid zurückgewonnen.

Beispiel 9

60 g (0,21 Mol) des in Beispiel 5 zur Addukt-Bildung verwendeten Phthalimido-methylierten Polystyrol-Perlpolymerisates werden bei 0 bis 20°C unter Rühren und Kühlen mit der Lösung von 37 ml (0,88 Mol) Schwefeltrioxid in 200 ml Dichlormethan vermischt. Die Mischung wird unter Rühren mit 58 g (0,63 Mol) Toluol versetzt und 1 Stunde bei 0 bis 20°C gerührt. Anschließend wird das Perlpolymerisat von der Reaktionslösung abfiltriert. Das Filtrat wird mit 300 ml Wasser extrahiert und der Extrakt mit 0,53 Mol NaOH neutralisiert. Die wäßrige Lösung wird zur Trockne eingedampft.

Es werden 93,1 g (89 % der Theorie) Toluolsulfonsäure (Natriumsalz) erhalten.

Die Aufarbeitung der organischen Phase liefert 5,7 g Ditolylsulfon.

Das Imidharz wird zur Herstellung des $SO_3$/Imidharz-3:1-Adduktes wiederverwendet. Da kein $SO_3$ mehr für die Sulfonierung des Imidharzes verbraucht wird, wird für die Addukt-Bildung nur noch die für die 3:1-Addukt-Bildung berechnete $SO_3$-Menge benötigt.

Beispiel 10

Es wird wie in Beispiel 9 beschrieben gearbeitet, nur werden statt der 58 g Toluol 29 g (0,63 Mol) Ethanol zugesetzt.

Es werden 75 g (80 % der Theorie) an Ethanolsulfat (Natriumsalz) in Form eines weißen Pulvers erhalten.

Beispiel 11

Es wird wie in Beispiel 9 beschrieben gearbeitet, nur werden statt der 58 g Toluol 67 g Xylol verwendet.

Es werden 116 g (89 % der Theorie) Xylolsulfonsäure (Natriumsalz) in Form eines weißen Pulvers erhalten.

Die Aufarbeitung der organischen Phase liefert 5 g Dixylylsulfon.

Beispiel 12

32 g (0,2 Mol) N-Methylphthalimid werden mit einer Lösung von 8,3 ml (0,2 Mol) Schwefeltrioxid in 200 ml Dichlormethan unter Rühren und Kühlen bei 0 bis 20°C vermischt. Die entstandene Lösung wird unter Rühren und Kühlen bei 0 bis 20°C tropfenweise mit einer Lösung von 17 g (0,1 Mol) Diphenylether in 50 ml Dichlormethan versetzt. Die klare Lösung wird 1 Stunde bei 40°C (Rückflußtemperatur) gerührt.

Anschließend wird die Reaktionslösung wie in Beispiel 6 beschrieben aufgearbeitet.

Es werden 35 g (97 % der Theorie) Diphenylether-4,4'-disulfonsäure (Dinatriumsalz) in Form eines weißen Pulvers erhalten.

Es werden 31 g (97 %) N-Methylphthalimid zurückgewonnen.

Patentansprüche

1. $SO_3$-Addukte von in Wasser schwer bzw. unlöslichen Imiden der Formel

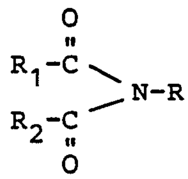

(I)

in der

$R_1$ und $R_2$ unabhängig voneinander für einen gegebenenfalls substituierten Phenylrest stehen oder zusammen einen gegebenenfalls substituierten Phenylen-1,2-Rest; einen Alkylenrest der Formel $-(CH_2)_m-$, in der m 2, 3 oder 4 ist; oder einen Ethenylenrest der Formel $-\underset{R_3}{\overset{|}{C}}=\underset{R_4}{\overset{|}{C}}-$ bilden,

in dem $R_3$ und $R_4$ unabhängig voneinander Wasserstoff, Halogen oder $C_1$-$C_4$-Alkyl bedeuten oder zusammen einen Butylen-1,4-Rest bilden; und

R für einen gegebenenfalls substituierten Alkyl-, Cycloalkyl-, Benzyl- oder Phenylrest, einen sich von einer Monocarbonsäure ableitenden Acylrest oder einen Rest der Formel

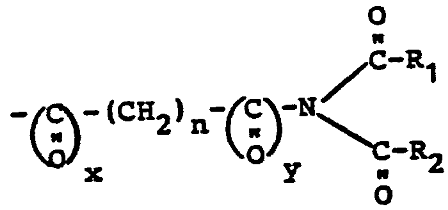

steht, in der $R_1$ und $R_2$ die vorstehend angegebene Bedeutung haben, x und y unabhängig voneinander für 0 oder 1 stehen und n die Werte 0, 2, 3, 4, 5 oder 6 annehmen kann, mit der Maßgabe, daß x, y und n nicht gleichzeitig 0 sein dürfen und daß x und y gleich sein müssen, wenn n 2, 3, 4, 5 oder 6 ist.

2. $SO_3$/Imid-Addukte gemäß Anspruch 1, dadurch gekennzeichnet, daß sie je Imidgruppe 1 bis 3 Moleküle $SO_3$ enthalten.

3. Monomolekulare $SO_3$/Imid-Addukte gemäß Anspruch 1, dadurch gekennzeichnet, daß sie als Adduktbildner Imide der in Anspruch 1 angegebenen Formel enthalten, in der $R_1$, $R_2$ und R die in Anspruch 1 angegebene Bedeutung haben, mit der Maßgabe, daß R nicht für einen gegebenenfalls substituierten Benzylrest steht, der an eine vernetzte Polyethylenkette gebunden ist.

4. Monomolekulare $SO_3$/Imid-Addukte gemäß Anspruch 3, dadurch gekennzeichnet, daß sie als Adduktbildner Imide der in Anspruch 1 angegebenen Formel enthalten, in der

$R_1$ und $R_2$ die in Anspruch 1 angegebene Bedeutung haben und R für eine $C_1$-$C_4$-Alkyl-, eine Cyclohexyl-, eine

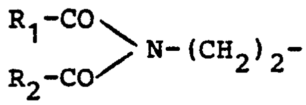 oder eine 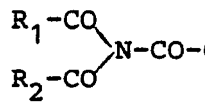-Gruppe steht.

5. Hochmolekulare $SO_3$/Imid-Addukte gemäß Anspruch 1, dadurch gekennzeichnet, daß sie als Adduktbildner hochmolekulare Imide der in Anspruch 1 angegebenen Formel enthalten, in der $R_1$ und $R_2$ die in Anspruch 1 angegebene Bedeutung haben und R für einen gegebenenfalls substituierten Benzylrest steht, der an eine vernetzte Polyethylenkette gebunden ist.

6. Hochmolekulare $SO_3$/Imid-Addukte gemäß Anspruch 5, dadurch gekennzeichnet, daß sie als Adduktbildner Imide der in Anspruch 1 angegebenen Formel enthalten, in der $R_1$ und $R_2$ einen gegebenenfalls substituierten Phenylen-1,2-Rest bilden und R für einen gegebenenfalls substituierten Benzylrest steht, der an eine vernetzte Polyethylenkette gebunden ist.

7. Hochmolekulare $SO_3$/Imid-Addukte gemäß Anspruch 5 oder 6, dadurch gekennzeichnet, daß die als Adduktbildner verwendeten Imide Imidoalkylierungsprodukte von mit 3 bis 12 Gew.-% Vernetzungsmittel vernetzten makroporösen Polystyrol-Perlpolymerisaten sind.

8. Verfahren zur Herstellung von $SO_3$-Addukten von Imiden der Formel

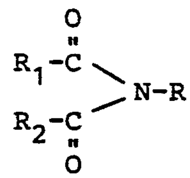

(I)

in der

$R_1$ und $R_2$ unabhängig voneinander für einen gegebenenfalls substituierten Phenylrest stehen oder zusammen einen gegebenenfalls substituierten Phenylen-1,2-Rest; einen Alkylenrest der Formel $-(CH_2)_m-$, in der m 2, 3 oder 4 ist; oder einen Ethenylenrest der Formel $-\underset{R_3}{C}=\underset{R_4}{C}-$ bilden, in dem $R_3$ und $R_4$ unabhängig voneinander Wasserstoff, Halogen oder $C_1$-$C_4$-Alkyl bedeuten oder zusammen einen Butylen-1,4-Rest bilden; und

R für einen gegebenenfalls substituierten Alkyl-, Cycloalkyl-, Benzyl- oder Phenylrest, einen sich von einer Monocarbonsäure ableitenden Acylrest oder einen Rest der Formel

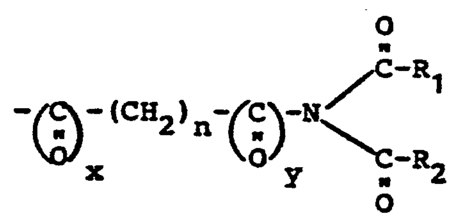

steht, in der $R_1$ und $R_2$ die vorstehend angegebene Bedeutung haben, x und y unabhängig voneinander für 0 oder 1 stehen und n die Werte 0, 2, 3, 4, 5 oder 6 annehmen kann, mit der Maßgabe, daß x, y und n nicht gleichzeitig 0 sein dürfen und daß x und y gleich sein müssen, wenn n 2, 3, 4, 5 oder 6 ist,

dadurch gekennzeichnet, daß man die Imide der angegebenen Formel in Gegenwart von gegen $SO_3$ inerten organischen Lösungsmitteln bei Temperaturen von 0 bis 80°C mit 1 bis 3 Molen $SO_3$ je Mol Imidgruppe umsetzt.

9. Verwendung der $SO_3$/Imid-Addukte gemäß Anspruch 1 als Sulfonierungsmittel.

0112541

Nummer der Anmeldung

Europäisches Patentamt

# EUROPÄISCHER RECHERCHENBERICHT

EP 83 11 2685

| EINSCHLÄGIGE DOKUMENTE | | | KLASSIFIKATION DER ANMELDUNG (Int. Cl. 3) |
|---|---|---|---|
| Kategorie | Kennzeichnung des Dokuments mit Angabe, soweit erforderlich, der maßgeblichen Teile | Betrifft Anspruch | |
| A | CHEMICAL ABSTRACTS, Band 79, Nr. 1, 9. Juli 1973, Seite 424, Nr. 4991c, Columbus, Ohio, US & JP - A - 73 10 024 (NITTO CHEMICAL INDUSTRY CO., LTD.) 08.02.1973 * Zusammenfassung * --- | 1 | C 07 D 209/48<br>C 07 D 207/40 |
| A | CHEMICAL ABSTRACTS, Band 88, Nr. 10, 6. März 1978, Seite 57, Nr. 75313e, Columbus, Ohio, US & JP - A - 77 106 819 (AGENCY OF INDUSTRIAL SCIENCES AND TECHNOLOGY) 07.09.1977 * Zusammenfassung * ----- | 1 | |
| | | | RECHERCHIERTE SACHGEBIETE (Int. Cl. 3) |
| | | | C 07 D 209/00<br>C 07 D 207/00 |

Der vorliegende Recherchenbericht wurde für alle Patentansprüche erstellt.

| Recherchenort | Abschlußdatum der Recherche | Prüfer |
|---|---|---|
| DEN HAAG | 07-03-1984 | MAISONNEUVE J.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82